# EUROPEAN PATENT APPLICATION

(11) **EP 1 149 915 A1**
(43) Date of publication of application: **31.10.2001**
(21) Application number: 00109218.8
(22) Date of filing: 28.04.2000
(51) Int. Cl.: C12N 15/63, C12N 15/82, C12N 15/11, C12N 5/10

(54) **Modification of gene expression in transgenic plants**

(71) Applicant: Frommer, Wolf-Bernd, 72076 Tübingen (DE)
(72) Inventor: Fischer, Wolf-Nicolas, Durham, NC 27705 (US); Frommer, Wolf B., 72076 Tübingen (DE); Hirner, Brigitte, 01307 Dresden (DE); Lalonde, Sylvie, 72127 Kusterdingen (DE); Okumoto, Sakiko, 72076 Tübingen (DE); Tegeder, Mechthild, 72070 Tübingen (DE); Ward, John, 72076 Tübingen (DE); Weise, Andreas, 72070 Tübingen (DE)
(74) Representative: Pohl, Manfred, Dr.

(57) **Abstract**

The invention relates to a regulatory element for the modification of gene expression in transgenic plants, to nucleotide enhancer and repressor sequences derived from non-coding regions of a sucrose transporter gene, and to suitable promoters for use in a regulatory element for the modification of gene expression in transgenic plants.

The object of the invention is to provide means for manipulating expression of plant genes, e.g. to control transport, storage, and growth processes in plants.

This problem is solved by a regulatory element comprising a promoter and a nucleotide sequence, wherein said nucleotide sequence is a nucleotide enhancer and/or repressor sequence comprising a non-coding region of a sucrose transporter gene.

SEQUENCE LISTING

<110> Frommer, Wolf B.

<120> Modification of gene expression in transgenic plants

<130> P423

<140>
<141>

<160> 9

<170> PatentIn Ver. 2.1

<210> 1
<211> 522
<212> DNA
<213> Lycopersicon esculentum

<400> 1
gtaattttca aatcgaaatt cgaaaattaa aattgtgttt gaacatatat ttaatcgaat 60
tattactgtt tttttttttt aactagctag ttgctatatt ttttatataa ttaaaatcat 120
ataaagacta catgcaatac aaaattccag ctagataaat gtaaaaaaat aatataattt 180
taggtacaaa caaaaaaaaa atagttctaa atatttgctt aaaaaataat taatggaatt 240
tcctcaataa ttattctttt ctaataacat gttttttaaa agatcaacaa ctttattctg 300
ttttttttaa taaaaaatta taaaaggtca caagttgtaa tttatttcta gtttcactta 360
gttatatata aaatttgatt gtttcgattt ttgatttaat tataagctca tgtgttacta 420
gattaataaa aaaaaagtca catgcagtga ttacagtgtt aaaatcaatc aacttttatt 480
actaatattt atgcgtcata attaaattgt ttttattttc ag 522

<210> 2
<211> 980
<212> DNA
<213> Lycopersicon esculentum

<400> 2

gtgagtttct ttccatttat tattacgata aataaaattt tagatagtca attaactgaa 60

ttaataaaat atttttttac aaaaaattta atatttatat ttagtatgtg tgaacatata 120

ttacaggtgg gttgactcat aagtccaaat atgacaaaga gatttattat gattttttaa 180

gaattttggt ttttttaggt tggtggaaaa tttatattat atggataagc aaagtgtgaa 240

aatattgacc aaatatgact ataacaaaca caaattattt gttagttata tacatgacat 300

cctcttcata tataggcatg taaaattaat taaatggttt tctttgtctt tgactatatt 360

tgaaagtaaa cattatatat catttttgaa attgaaattg gatatttagt tatatcattt 420

ttaatagcaa ttaggatgaa cattagccac atgtatgttt tcaatggata atcaaatttt 480

tttttgaaga aaatttaaag ggagtatagg ttatggcttt taccataaat agatcatgat 540

atgtatgtgg gcttgtaaac ggttgtaagt cacacacata aatgtagaag gtgtgagaag 600

ctttttatta atgcatcaca tgacttttta gttttgttaa tttctagtta tttaaattaa 660

taattatttt atgaataaat aattttaata aacatgacaa ttaaaaagaa tcgactatta 720

tttctcttta tcctaacata aggattagga gaatcttatt actttttgat taggataata 780

atacaagttt ctatgcaaaa tcatctttta ttattattat tatggcaaaa gcatatatct 840

ctcttcttct tcctttaaat tattctttta agatagaatt atttcacttt tgatgtttag 900

attttccctt tttatctttt ttggcaaatt gatcatgttt ttttactaat atatgaaata 960

aaaataattt tattttacag 980

<210> 3

<211> 684

<212> DNA

<213> Lycopersicon esculentum

<400> 3

gtactattta atttttaatt cctcaatcat ggataacgta cttaattctt ctttttaatt 60

atcttacctt tcataaatta atgcaccata tatttaaaca caaacgtgta tctaaatagt 120

gaaaaatata taaatgctag tctattacgt gtatatactg ttatagatcg aatttatgta 180

cgaaacatta ccaatcaaag caaatgtagt atcgttttca aactattgac agtcttgtag 240

ttaactaatt aaatttagat ctccatccta caacgcaagt tgtaaaaaca cctcgtaaat 300

ccggacacat gttgtaggat taggggtaga tgttttcgag gctattaata gtgtagagat 360

aaaagttttg gagtgttttc aatacatctg tctcgttaga aaaaggtcga ggcccacgtt 420

aaccggccat tgtcacctag acccctacaa taaaaaaaga aaaagaagaa caacgtataa 480

ataggggaag atcataactg aaggaatttg aaaatgacag aactagaact acattaatga 540

gatatgacca caagatataa tcttatgact atacttctta ttattcacat taggataaac 600
taacatacta ttattgatat taaattagtt ttaaatacac aaggacctaa ctataactac 660
ttgttttttt tttttatgat atag 684

<210> 4
<211> 1231
<212> DNA
<213> Lycopersicon esculentum

<400> 4
aaaaaattac aaaagacgag gaagaagaag aaggagaaga aacaaacttt ttttttatat 60
attagtactt ctcttttgta aacttttaga aaacaaacat aacatggagg ctatcatgtc 120
catgtatctt cctttttttt tttcttttca taaagctctt tagtggaaga agaattagag 180
gaagtttcct ttttaatttc ttccaaacaa atggggtatg tgtagttgtt ttcaatttgt 240
gtgtaacaaa gaaagttgtc cagtattaac atgtctgggc aaatgctttg taccctttta 300
ctatttttat ttttattttt ggtaattttc ttcttggaag gtaggaaaaa aaaaagaagt 360
ggttggaggt ggaagtgggg tactttaatt tgggttggta agaaggggag atttatgaaa 420
aactttgtac ttaaagtgga atcaaaattg atctatctat atatctatca atgttttcta 480
tttttttgtt tcttgtttca accattattt gaactttttg gtggattgat agcactattc 540
ttaccttatt tagtggtatg catcttctag cacttcccaa actttgaagt attaattaat 600
aacaacatta atttgtaata tacttgtatt atattgagtg gtggagctac gatactaaag 660
ttagtctact atgctctcaa agagttgtta ccagtgagat ttgaacacat aaatacttta 720
tatttgtgct aataacctgg tttggaagca acaaatcaca ggagcgtagt ttcttttgtg 780
cttcggggaa gaggctgctc agctcctata aaagctaatt ttgattggta tcattcaaat 840
gcgaatatag gcctagtcgc attgctcttt taagacgctt gaaacaattg agttacatca 900
tattcttatg tcaagaatgt cccaaatatg aaattacttt agttattgta ttacttgtat 960
ataatgtata atttttcgac aaagggtcta taattgaaca tcagtgcaac tacgccccta 1020
ataactattg gaaatttgtg tttgttttac catcctaaat actagtgcac taattggaat 1080
aagttatata taaatatttt ggaataatat tttctattca ttacaaaatt aaaaacaaaa 1140
aataacaagg ttttttaaaa cacttatttg aacaaaattt catgcgtaaa ttgaaaggga 1200
attttactgg agataaatag gatttgggag t 1231

<210> 5
<211> 2354

<212> DNA

<213> Lycopersicon esculentum

<400> 5

gaattcttgt gtatcatatg tgagatataa aagaaaatga attttttttt atttaaaaaa 60

aaaaacgtgt caatacgatc tttttctagc tagagcacca ttttcaatta attttacaaa 120

aatataaaaa aataaaatat aaaatgaaag tagtaaagaa tcagagttgt ctttatgttg 180

agacacaaaa atttatgaaa ttttttaata cataacataa aaggggaaga aaagctgcaa 240

cagtgaattt aatgataata aaacttactg tatgtttttc tggtcctata tttggataag 300

tctccacttc tactttttcc ctttaattaa ttcttgacta gagagtttaa atttgtaaaa 360

ttataactat gaattgactt ttgaaagtta ctttttcttt ttcaatttat tacttattaa 420

tatgaactct tgtttgtttt gttaaaacgt aattacttat gttagagaaa atattttata 480

tgaatgtggt actttttata tgactttaaa tttaattggt tttgatgtaa atgtcaacta 540

tcacaattca tatgtaaaaa aattttaaaa aaaatattat ttatcataag aaaatttatt 600

ttggatccta ttatatctta attgttatcg ttcacttata tctttaacta tattaaaaat 660

aaggatgaat tacataaatt tactagttta gtgttgatta tttagataca agtttattta 720

taatattacg tatttactaa agtttggtgt gtttgataca tgtgtctagg caaatatgtg 780

gtcgaaattt aagtataagt tgttctagat ataatgcatc cagttggtga attcgcatgt 840

accatgataa tatatagaca aatcatgctc gcctcctttc catctcattc atcgctcttt 900

tatcttgctc gcatatatga agtgattcgc atgtgtatga tatacacacg taaaaagatc 960

tcgcttgcat ctctcgtttt ctcacataca tatacatata tatatatata tataaaacga 1020

tcttgcttgc atctctcgtt ttctcgctcc ctcccttcct atatctacta gcaaaaaata 1080

tgtatgtaga tatatttgat ttgaaagttg ggggtgaatt tattaagact attgggataa 1140

aatgctatta taaggagaat aaataaatat gattaataaa cattatgcat ttagatagtt 1200

tcgcgtaaaa ataaatatat cttgttattt atttaatttc aaaaattaaa aaataattca 1260

gaacgctttt gttttcctca ctcctcaccg tccatcagga cacttctact ttcctcaatc 1320

ctcaccgtcc tcggcttgct tacgtagtaa aaaataaaga aattgtctat gacagatgga 1380

atacgagatc taggtaagtc tttctttttt tgtcaacttt aatgctacgt agactttatt 1440

ttattcttat ttacccttat aataaatatt agttataaac aaatttgata tctcaattgg 1500

ttgactacct gaacatttat cttgttagca acgattcaat ttcttatttt ataatttttc 1560

gtctcatttc tcatttcccg tgttcccatt atacttgcaa taattaaata taaaatgtaa 1620

taaacttaat agattttttc atataatttt ttaaaaccga tataatatta tttgtggaag 1680

cttgaaagaa tgataaatca aggtgatata tgtacatact agatacattt atttttccaa 1740

acatgtgaaa ccactaccaa tctagcattc aatagaattt cctaacaata aacaatattt 1800

gaaataacga aaaaagacaa aaactttatt aaatgataga agcgtttcgc ttttttagaa 1860

ttttttctac ttttattacc caaactaaaa tttcaaaatc tacttttccc atttaaataa 1920

tttcattctt aaatcctttt atttattttt ttaatataac tgggtctggg gtggcaacag 1980
tgggggctgg ttcttaaaaa cctctgtggc cggtcactct atcgttctca aatttcactc 2040
gtaaaaattt cattgaatat attattagtt caatacattt taaatttgat aaaagatcaa 2100
atcagatagg taaattgaaa tggatgaggt aattaaatta tttatgaaaa atacaaaaaa 2160
atttattact tttgactaaa taaataaaaa aaggatatta ttctaccaca tgcatgacta 2220
ttgcctccaa aaaaacacta taaatacccc cttccattat cccctttctt tataacattc 2280
aaaacccaaa attaaaacag agcaatttta gtttcttaga agaaaaaaaa atggagaatg 2340
gtacacccgg gtac 2354

<210> 6
<211> 3100
<212> DNA
<213> Arabidopsis thaliana

<400> 6
cctagtctcc gacttaagac tcgagatccg gtctttctgg tggcgcgtga ggtcggccac 60
ggagacctcg gtgagtttaa acacaagaga aggcttgaaa tcgccgaccc ataaaataag 120
tttagcgtag gagttaaacc acggtgggca gaagaaagtg aaaacgttgt ccccggcgag 180
ggaaacggag gcgaatttct cttggtagta ttgaagacag tgagatagaa actgggccac 240
gagagattgt tgttcttcga gagagatacg attagtttca tccaaggaag aagcacacat 300
aagctgttca acgaaatacc tgtgacggat taaccaacca tcaacgaaga tagtgaagct 360
ttgagagctg ctagtgtttg gcatgtttct tatgtttgga gtactcacta agtaagtatg 420
gactgtgagt gtacgttgtg caagaatgtt tatatactac tacattgggc ttgaccaaga 480
aaaatctttt aggaattaat ttacgatatg tcggtctatc gacgatgtgt cggcgggcga 540
aatggatttt tgtgactttt gtcttgtcgg agtgaagtgt aatcagagca gcgtcttatc 600
atcatttgct gttgttattc aactctggtt ttagaatcaa gttgggttaa acaatagtat 660
aaaattttaa tatggatgaa aataaaatat aaattatcct tggtaattat tgattataac 720
ctatagaatc gacaaaaaga aagactataa cctagagaat tttttttttt ttttaaaaca 780
agtgttcatt aaaaatcttt aaaatattta gtctgtttta taattaaaat gtttatatct 840
gaaattgtat ctatcactac tagttccagt tggaattata tatctgtcag ggtcgcatct 900
cgatattatg gaatgaccta aaataacatc tgaagaggaa accgacacat gttggtaaaa 960
taagaaggca cacgtcatgt aaagaagatg acatatgtca ggaaaggaac atgtccttac 1020
aaaatagaaa ccaaataaac caacagttca taaatgctcc agttatgccg cgagccatct 1080
tttcttcttt actaagaaaa tttctacatt attttaagat ttttcttttc catgtagttg 1140
tctcatactt ccttcgaaaa gatctttctc taacttcaac cttggggatc gagcaaccat 1200

atttcaatca acagattctt tcatcactct aataacggac aaaaaaagaa aagatgtcaa 1260
aaaaaaaaaa attggaatat gcacgttgat actataagtt catttaaatc agtcgttaga 1320
gtaaacctta ttgagagttc aaattcattt aaaaacctga tgagataatt atataatttc 1380
ttataagatt tttttttctt gtaagaattt aacgaataga atttttctct ctattactta 1440
aaattcctcc tcgtcaaaaa ttgcaaatgc ttggcagttt taccattttc tcagaactac 1500
cgtatgagaa ataaagtacg tattaaaaaa caatacaagc tttaaagata aaagaggcaa 1560
caaatttttt tgacaaaaaa attagatgaa aagaagtgac gacaaataac tgagaaaaca 1620
cgttttgcaa aactaaaaaa aaaaaaaaaa agagagagtt ggaaaatgtt tggagctggt 1680
gggcattagt tggctatgca acgcacggac gcggatactt ttcaactcgt tgcttacgcg 1740
gccacgtttt cgtcacaaac cgtgtttctt ttgcggcctt ttaagtttta atcaaacatt 1800
tttgttttca tttgaatctt ttttttaagg cggttatacg aataaaatca ttaaaaggag 1860
aaacgggcct tcatgtttag gtttttgttc ttcctcattt gggttattta gcttattggt 1920
tcagcccaca agagtttcct tcaatggata gggccttcag ttgcatgcca tcatcctttc 1980
ttgtttctct ataagccatt tttgcaatga gaatttctca atttcttatt tttgcaataa 2040
aatcattttg ccttagatga tggtggaatc atccgttttt ctttattttg acccggtgag 2100
taattgaacg caaaaaatag tccaattatt caaaacctta caatttcata tcgccataat 2160
aaacctaaac atgattcgag tgagcaacta cataaaaagg ggaacgaaat gttggtacaa 2220
aactgaattc caacccaatc aagtgcatac taaactattg agcaaaaaca aagataaaaa 2280
tcagttcacc taccctaatt aaaacatgat taggcctaaa agactataag taaagataaa 2340
aaagatgcca ttttagagta cgttttggta aattggcgcc atcgtgttat acgtttaaac 2400
cgtataacct gaatcatgca gcacgtgtac ggctcacatt ggcccaagct tgattaacaa 2460
acaataacgt cattttcgta tttcaacaat tttgatcgct caatcctttc gacctaattt 2520
ccatattaag taatttatta gttaaaacaa cggactcaac caaattacat attaagagaa 2580
tatttgaaaa ttgtctatgt agatgaacat gtatttgaaa attgcgtaag cacactcttt 2640
ctctctctct tttttttttt tttttttccc cctcttcttt ctttgaataa agatgacatt 2700
tttttctgtt ccaaacacaa aactgcatat tgatagtgtt attgaatact caacgtctct 2760
tcttccagct tacgtaagtg gaacttactt tttgccccgt gatccaacag aaaatgacga 2820
atgctccctc aaaatcattt cgtttcaaca aaaatatgta ttttatccat agagaaacaa 2880
caaaccttac actatatacc ctcttttaac gtgcccactt ttcattattt acacaatttc 2940
cccaacaaat ttaaaacaat ttacaaaaag cactcaactt tcctaaagta gattccttta 3000
gtccaatgga attcttacaa catcttcttc ttcgccacct ttttctctcc actttaagct 3060
ttttcactct ctctacgcca cgacgactgt gaagcgagaa 3100

<210> 7
<211> 2441

<212> DNA
<213> Arabidopsis thaliana

<400> 7
taatgattaa tgaccattat aatttttctt tatcaacttt aatggagtta ttatgtgaaa 60
cagtgtaagc cccattctta tgtatatatg taaatatcac attataaatt atgatctatc 120
gacagagaac gacaaaattg ccaaccaatc actaagttag aaaattaaat ttattgtaat 180
aacgcatctc tttttttttt tttaaacata tagtttttat attattaata tttgaatttg 240
tcgtttgaga aatgacgaag gagttatttt caggcgtaag catttgacgt gctcgacgtt 300
aaggagatga cgtgttaagt tatggtacat atgttttttt ctcttttatt tgggtcatag 360
ccgcgttcaa cacgtgcttt ttgtcaaatg taattacatg attaaactaa aaaaacttat 420
tactgttttt caatcaactt attaaccttg atctttttta taacaaaata aaaataaaaa 480
ttggcacatg atcaaatcat caatctcaaa taagataaaa ccaaaggtgt aataaggttc 540
atttttttgt ggagaaaccg agtcaatgct accaaattgc ctatataggc ttgaatttta 600
tgtaaatgct tttttgaatg gaataatccg attttttatt ttattttttc ttatgtaaaa 660
ttataacatg gtatagataa aaaatttgtt ttgtaataag tgatatttgg tctaacggcg 720
cgtatgtata gttcttttac aaaagtgagt aatagtggtt ttctggttca aatactattg 780
agaatggtat cagttttgat ggacaacaag tgtatgagac tcggctataa cttctcaatt 840
aaccattaac cattaaagaa tacaaaattt ggattttcga ctatactttg gttcggatac 900
atcatacatg catgcacacc ataacaacta aactcaaaag gatttgtttt tgaacttttg 960
atgataagaa aaagaaaaaa aagatgggac ataagagtca tgtacacgtg gataaggaaa 1020
ggagaagaga gggacaaggg aagagatgga gaaagagaga cgtaagtatt aattcaattg 1080
tgtgccctat tagctttcat tttccacaag caagaataat attaataatt atgtatcatc 1140
tttgcatttt tgtaccaata catgatacat ttgttaggca cacgtctttt tcctttttcc 1200
tcctttttat ttctaacttt cttcctttta ttcgtttaat ccggctctat cacgttaaca 1260
cacatatatg ttatatcatc tttttacaaa catgtttaca ttagttatta gaaaatacat 1320
atacttgtaa tagtgacatt ggtaagaaga actctatcac cttcacaatc acacacatat 1380
atgttatata atctttttac aaacatgttt acattagtta ttagaaaata catatacttg 1440
taaatagtga cattggtaag aagaaaaaaa cactattaaa tagtgaaaaa atgtttataa 1500
ctctcttaat taacattact tattattgct agcacctaaa atctcccaca aaatatttgt 1560
tgtaaaacac aaatttacaa aatgattttg tttttaaatt agtaacacat gttcatatat 1620
acgttaataa gaacataccc tatatgattt tatataaaaa aatttctttg agacgtctta 1680
tccttttttc tttaataata tgcaattgtg agagtttgga tttgaatggt agcattagaa 1740
gcaaacttga accaaacata tttcatgaag tcaaacttga accaatgtaa tcactaatca 1800
cagtgttcgc agtgtaaggc atcagaaaat agaagaaggg acatagctat gaatcatata 1860
atcttgacac atgttttata ggttttaggt gtgtatgcta acaaaaaatg agacagcttt 1920

cttctaatag acttaatatt tgggctaaat gtaccacagt tgtgaatttc ttacaaaaat 1980
gggccgagct acaaaaaact acaggcccac tctcaactct tatcaaacga caccgtttta 2040
cttttttaaa agcacacact ttttgtttgg tgtcggtgac ggtgagtttc gtccgctctt 2100
cctttaaatt gaagcaacgg ttttgatccg atcaaatcca acggtgctga ttacacaaag 2160
cccgagacga aaacgttgac tattaagtta ggttttaatc tcatccgtta atctacaaat 2220
caacggttcc ctgtaaaacg aatcttcctt ccttcttcac ttccgcgtct tctctctcaa 2280
tcacctcaaa aaaatcgatt tcatcaaaat attcacccgc ccgaatttga ctctccgatc 2340
atcgtctccg aatctagatc gacgagatca aaaccctaga aatctaaatc ggaatgagaa 2400
attgattttg atacgaatta gggatctgtg tgttgaggac g 2441

<210> 8
<211> 2192
<212> DNA
<213> Arabidopsis thaliana

<400> 8
gatcttaact tttatttaaa tactactatt acagatcatt atctaaatag aaacctaact 60
gataatagta agcaacgaat ccgatgcctc ctctttctct aaatgatcga atgccgataa 120
atccgccggc cgtcctgaaa agtgaaaagg gagaatgtaa gtgttaggag gatatagtac 180
tcatggcgat gtatactctt acaaagataa taaaaataat aatgaagata actcttacta 240
agggatcaag ttgaagaaag tgcagttgct atgatgaaag ggcggtttct cattcatttt 300
tttaattcgt taatcgacat tgaggatggc tgtgtgtgta ttgtgtgcgc ttaaatcaag 360
tggatgatct taaccaaaga accatattct ttttcatgat ctcctagatc tttgttatta 420
cttttaatta tcttttcaat gtaaacatgg aaaaaaaaaa aaactggtac attttgtatg 480
catttttgcg gttataaact ttggaattgt aaagaatgaa ctagatttta acctgctgac 540
accgagacaa tttatttttt taaattagta tatataaaat tttgcaaatt atatctatct 600
ataaaatatt tttattttat agtttataat tattattaag taacatcctt ctaaacccgt 660
tccaccaaac tcgtcctgta aaaaaaaacc gcggtacccc gtaatttaaa tttataatta 720
aaataaatat ttacctaggg attgcaattg tgtttagttt gtcaaacaaa attttgttta 780
aatagttgtc gaaatctagt tcaaattttg gagacataag atttagtgaa tagataagaa 840
atgtatttag ttgcgttgct cattttaagg gattgcaatt gtcttttggg ttgtcaaaca 900
ttttttgttt taaatagttg tcgaaatcta ttttaaattt tggaaaaatc tgcataatat 960
atgggattgg aatatatttg gaatgttttt tcatttgtta atcaatttat tgctgaaaga 1020
aacaataatt aaaagccaat gacagccatt gtaaataagt tccaacttca ggatttattt 1080
cacaaaattt cacaaaatga ctgcaacaat gtatatataa attctatttt tatgaatata 1140

attccctttc tatttggaga ctattttctt tgaatgatga cttgtctgaa tacaaatctc 1200
ctcgtaagtc cttaaaggat atttggtttc ccacgaatca agccaataca agtttgtgta 1260
tgtcatttat ggtttaaatg agtttagttg gcaaagtaat caaaatattt catttcgaga 1320
agaacatata gcttacacgc ctcataagtt agttacctac agaaacttat atggtggggt 1380
gatatacttt cggattctag gtaagatata gtatatgtgc gttactattg ctcatactat 1440
aaaaatcacc ttttggtttt gagttatcat tttgaaatct acacatgatt gcgtgtactc 1500
ttatttaaag tcggcataga tttctccata tatatgtcat gattccatga atgttctttt 1560
aacatcttaa tgcgatttta cattaaaata ttcaagaaaa aaccaagctg ttttctaaat 1620
aaatttagtt tgtccacggt taataaaatt taaaattagc gaaatacgta aataatcaaa 1680
tgatataacc aaaatgccta ttatttttaa aacaacattt tattttctct aatctcataa 1740
aatactctaa taaaatctag ttgtaagtga aaataacact aagcttcaaa cacacacgta 1800
catgaatgtt tgtttttttc tttcaaaaaa gctgaacaat gacattttaa gtccaaatga 1860
aaacgctggt atcatcttgg acttccaatt ttccattgcc catattttct cttttctttt 1920
gtttttatcc aattggtatc gaatcattca tataaaatta taaatcatga agtcaaattt 1980
ttcaatagta acacttgagt aatagtggtc ctaccaactt tttaccaaga cagatatatt 2040
ttttttaata cactttttac cttttactgt gtttgcctca aatgagtgtt tttaccagtt 2100
tctgtgtata taaatctgca ctccactctc agtttatctt catccacaag acagttcttt 2160
aacaaagtca ttaaacattt cccttacctt ct 2192

<210> 9
<211> 2694
<212> DNA
<213> Arabidopsis thaliana

<400> 9
gagattgaga tgggacctct gcgaatgggt gggcttgtag cttcctcggg tacaaaggtg 60
ttgactatcc gcaaaccggg tgttcgagtg gaccaagact ggaccgtaaa gattctgtga 120
ttgaacggtt tgaaccagtt tcactcatgg ccgttagagt ggccgaaatc tgcttttccg 180
gcgacggaat atcacacttt ttaatatatg tttggagatt tagacttaaa tagttgtaag 240
agctaacagt ttgaaagtca ctttgcattg ttgtttatct tcatataaat gagtttagat 300
tttgataatt tcagaattcg tggaatcata attaacaatt ttgataggga aaaataattt 360
gtttttttta gtcagagggt caaataatct taccttccat cgttgcctaa ttattgtcaa 420
atccttttac tttttaccta atcaacccat gaaaaatctt gaattacacg ttttaagagt 480
catgaagaaa aatcaatctg attctgttgg ctagatttgt tttttttata ctgcttagtg 540
tttactgtta tagaaagggt tctaagtatg caaagtttgc cctaattaga taatgttaga 600

tccgtgtagg tgaaacaaga agccaaaatt ctgtgtaaaa agagtcacgt aactacctaa 660
ctcttgtagg tctaaagacc atcttcactt taaattagaa cctcatgttt cccaaacaat 720
atattccgtt catgaatctc gtttcgaatt aaaactaaaa tattacacat tttagaatgt 780
aagaaaaaca cactcaaaat taagaatctt ggaattgaaa acgagatttt gaatgtgttc 840
aaagatactc tttagaaatt ataatggtag ataatagcac ttgcatgaca aattcgattc 900
tctcttctca tcatgcattt ttgctttaca ttttgttcct ttaaaccaag aatattttga 960
ctcacctaaa agataaacca aacatatctg aaatctacaa ttgacaatta acttctcttt 1020
ttcttttctc ttattcttac tgaaataaaa atagtatgca actggtcttt ataaatttca 1080
gttttatata tttaaaaaaa atcttctata ccaagtttac caactcatgg tagtccaatg 1140
attaaaaaat aaatcattat ggggttagga cttaggagat cgaattcgaa tcatttcaaa 1200
ttttcaatgc gatttcaatc attgtttttc actgttaaca ctttgaaata tctaagtgta 1260
tctaaaaatt tcaagcctta gagattaatt tggacttcga tccggaattc tgcgacgact 1320
atcaaacaaa ttttgtttta accgaaatat agattatttg catctctgat tatcaaacca 1380
ttcattcttt ttattattat tcagtttact ataaagtata tgtccctccc tttatgaacc 1440
gtatcaaata ctatacatag tatgcatgtt cgaagaaatt tccaacatac taatttgaaa 1500
tcaacgaaca tatcaaatta taatgcgttg atgcagaata tatgctttct atatatataa 1560
cttaaataca aaaacctaaa ttttccatat gccttcgtaa tttagtatat aaatattcat 1620
tcattcttaa tacagcctag gactcggtag cattttgaag taggaacgaa ttttgttaat 1680
taagatacat gagtgattag atcgaatcca aaaggtatgg aggcgtagaa caagaatttg 1740
gttctctttc attttacgta tctttcacta aagaatagag agaaaacaaa accatgcaaa 1800
aacctctcaa aaattatata ttgaacgctt agacaaagag ccaaatttat tcgctttttg 1860
atgtttacat cagtatttgt ttctagctag ttcatattat ttaaactcca taatcatttg 1920
aaagacataa aatacagatg tttaagatct gtggatgttt atataggatc ttcgaattcg 1980
gataatgaaa tcatttatac actattaagt attaactttg tagaggactt aagtattcga 2040
aatcttgatt caggttgtca atcaagaacc gtaatgtttg atctatagag ccatgggatt 2100
aatggatgct aaaagaataa aacactcgag aagaagagta cacgtaaaat cactcacaca 2160
caaagaatga gagatgggag aagaatacta caaagtgttt gtttttttta aatcggctca 2220
tatataaatt tgggcgatac tctactaccc acattattct tttaaacaat gatataaatg 2280
tgtatggcct atgtagccga actttttaca tatctgacct gtatatatat aataattatt 2340
tatcaaagaa attagagaaa gattcttcca cttttgatgg cttataaaag atgatgaccc 2400
aaaacactca aaatgcagca gcaactgctc tctcaccacc catctcctca tttctctttt 2460
gcatttcttt ctttttttct gcattgcatt cttttgaggg gtttaatttt ctgcatagct 2520
ttgtctaatc tcttagagct caataagaga aggtactata actgatctct ccctctttca 2580
agtttttttt ttgtttggtt tatagaaaac tataaccgcc attatccgtt agttttaacc 2640
gttttttgaa actagtgaat cctcataact ttggtttctc actaaaaaca gatg 2694

## Description

The present invention relates to a regulatory element for the modification of gene expression in transgenic plants, to nucleotide enhancer and repressor sequences derived from non-coding regions of a sucrose transporter gene, and to suitable promoters for use in a regulatory element for the modification of gene expression in transgenic plants. The invention further relates to vectors, host cells, plant cells, plants, seeds, flowers, and other parts of plants, comprising a regulatory element for the modification of gene expression in transgenic plants.

Plants use a vascular system, the phloem, for long distance transport of assimilated carbon compounds from photosynthetically active or storage tissues and organs (source tissues/organs) to non-photosynthetic parts of the plant (sink tissues/organs), where these compounds are consumed. Main components of this transport system are connected cells, so-called sieve cells or sieve elements, and their companion cells. Sieve cells are enucleate in higher plants and are kept alive by the companion cells, which produce micro- and macromolecules and translocate these substances via plasmodesmata into the sieve elements. The sieve cells form the conduits for long-distance transport in plants. Therefore, substances synthesized in companion cells such as proteins, peptides, RNAs, and other products of enzymatic reactions can be distributed from source to sinks via sieve elements. Substances that are transported into companion cells or sieve elements also can be distributed from source to sink via sieve elements.

The disaccharide sucrose is the major transported form of carbohydrates in plants. It is synthesized in green leaves and is transported via the phloem to support growth of sink organs, such as roots, meristems and flowers, or to support storage, e.g. in tubers. There are three principal locations in plants where sucrose uptake transporters in the plasma membrane are thought to be crucial for long-distance transport. First, in most plants, sucrose must be actively transported into the phloem cells in a process called phloem loading. This loading process is supposed to create the driving force for long-distance transport. Second, sucrose transporters serve in re-uptake along the length of the phloem to prevent excessive losses during transport and to recharge the driving force continuously. Third, in sink tissues such as meristematic regions and storage organs such as tubers and fruits, sucrose transporters are responsible for sucrose uptake by sink cells.

Sucrose transport at each of these locations is mediated and regulated by a set of specific plasma membrane proteins, the sucrose transporters (SUTs). Sucrose transporters are further required for sucrose efflux from cells located near the phloem in source leaves, for sucrose efflux from the phloem in sink tissue, and for transport across the vacuolar membrane.

The first sucrose transporter, SUT1, was cloned by functional expression in yeast (Riesmeier et al. 1992, EMBO J. 11: 4705-4713). Related genes from plants have since been obtained using the sequence for SUT1, including three genes from tomato (*Lycopersicon esculentum*). LeSUT1 and its orthologs from other plants are hydrophobic proteins consisting of 12 membrane spanning domains and are located in the plasma membrane of cells mediating highly specific influx of sucrose using a proton-coupled mechanism. The use of transgenic plants specifically impaired in SUT1 expression has provided strong evidence that SUT1 function is required for phloem transport (Riesmeier et al. 1994, EMBO J. 13: 1-7).

Transport of amino acids is another process that is of high importance for the growth of the whole plant and plant organs. As amino acids are indispensable for protein synthesis and other purposes, e.g. as osmolytically active compounds, limitations in their transport via amino acid transporter/permease (AAP) proteins will result in a limitation of plant product yield.

The above mentioned transport and storage processes need highly specific gene expression. If means were available to control expression of genes involved in these processes novel transgenic plants could be produced with desired advantageous properties e.g. enhanced growth, increased yield, resistance to pathogens, larger leaves, and sweeter fruits.

The object of the present invention is therefore, to provide means for manipulating expression of plant genes, e.g. to control transport, storage, and growth processes in plants.

This problem is solved by a regulatory element comprising a promoter and a nucleotide sequence, wherein said nucleotide sequence is a nucleotide enhancer and/or repressor sequence comprising a non-coding region of a sucrose transporter gene.

The regulatory element according to the present invention, comprising promoters and nucleotide enhancer and/or repressor sequences, can be used to influence the expression of native or introduced genes. These genes can be of plant or non-plant origin.

In a preferred embodiment of the invention, the nucleotide enhancer and/or repressor sequence is derived from tomato (*Lycopersicon esculentum*).

Unexpectedly, it was found that a nucleotide sequence from a non-coding region of a SUT gene, especially a nucleotide sequence of intron 1, and/or intron 2, and/or intron 3 of the SUT1 gene, alter gene expression in a way, which is suitable for achieving overexpression or decreased expression of genes in specific cells and tissues.

Thus, a new regulatory element according to the invention drives high expression of genes, stabilizes their mRNA, or decreases gene expression. This can be used to
1. alter expression of sucrose or amino acid transporter genes (more phloem loading at sieve element level, more retrieval along the transport path, more uptake into sink tissues) by expression of SUT or AAP genes in sense orientation.
2. alter the expression of other genes in companion cells, guard cells or trichomes
3. introduce the expression of plant or non-plant genes into companion cells or guard cells or trichomes
4. introduce compounds into the phloem such as RNA and proteins. These can be either new compounds, e.g. from single step pathways to generate e.g. octopine, regulatory RNAs or proteins. This may also be used to trigger systemic acquired suppression to create virus resistant plants or to decrease the expression of target genes
5. introduce or alter the expression of genes in the trichomes to increase plant defense against insects and herbivores or produce molecules with industrial or agricultural value. For example, the expression of monoterpene biosynthetic enzymes in glandular trichomes could be modified. Also, the production of vacuolar flavonoids produced in glandular trichomes that have antiinflammatory or other pharmacological properties could be manipulated.
6. introduce or alter the expression of genes in guard cells. Natural or synthetic receptors, signal transduction components, ion channels or transporters expressed in guard cells could be used to modify and/or allow control of stomatal aperture regulation. This could increase drought tolerance in dry conditions or increase CO₂ fixation under conditions with sufficient water.
7. introduce xenobiotics into the phloem. Since many compounds applied to plants (xenobiotics such as herbicides or pesticides) are glycosylated, overexpression of a transporter for xenobiotics in companion cells will allow better mobilization of these compounds
8. modify companion cell activities, e.g. enhance ATPase activity and increase raffinose biosynthesis
9. block phloem loading to increase carbohydrate in the leaves e.g. via cosuppression or antisense suppression

Intron 1 of the SUT1 gene decreases gene expression and is considered a repressor of gene expression. Two other introns enhance gene expression and influence the spatial distribution of gene expression. Intron 2, for example, enhances gene expression in guard cells and phloem veins, whereas intron 3 enhances gene expression in trichomes.

According to a preferred embodiment of the present invention, the regulatory element comprises intron 1 of the SUT1 gene.

According to a further preferred embodiment of the present invention, the regulatory element comprises intron 2 and/or intron 3 of the SUT1 gene.

According to another preferred embodiment of the present invention, the regulatory element comprises the sequence of SEQ ID NO: 1, or the complementary sequence of SEQ ID NO: 1, or hybridizes with the nucleotide sequence of SEQ ID NO: 1.

According to another preferred embodiment of the present invention, the regulatory element comprises the sequence of SEQ ID NO: 2, or the complementary sequence of SEQ ID NO: 2, or hybridizes with the nucleotide sequence of SEQ ID NO: 2.

According to another preferred embodiment of the present invention, the regulatory element comprises the sequence of SEQ ID NO: 3, or the complementary sequence of SEQ ID NO: 3, or hybridizes with the nucleotide sequence of SEQ ID NO: 3.

In addition, a 3'-untranslated region (3'UTR) of the SUT1 gene further contributes to the level of expression by stabilizing the mRNA. Therefore, according to a another preferred embodiment of the present invention, the regulatory element further comprises the sequence of SEQ ID NO: 4, or the complementary sequence of SEQ ID NO: 4, or hybridizes with the nucleotide sequence of SEQ ID NO: 4.

Nucleotide sequences of intron 1, intron 2 and intron 3 are given in the sequences with SEQ ID NO: 1, SEQ ID NO: 2, and SEQ ID NO: 3, respectively. A DNA sequence of the 3'UTR is given in the sequence having the SEQ ID NO: 4.

Genes coding for transporter proteins are active in specific regions of the vascular system or in specific tissues/organs, respectively. Gene expression and spatial distribution of gene products depend on promoter activity. Consequently, the use of tissue or organ specific promoters within the novel regulatory element according to the invention allows systematic manipulation of transport, storage and distribution processes for assimilated carbon compounds, e.g. oligosaccharides like sucrose, or amino acids.

Therefore, in a preferred embodiment of the invention, the regulatory element comprises a promoter, which comprises a nucleotide sequence of the SUT1, SUT2, SUT4, AAP3 or AAP4 promoter, or a complementary nucleotide sequence of the SUT1, SUT2, SUT4, AAP3 or AAP4 promoter, or hybridizes with a nucleotide sequence of the SUT1, SUT2, SUT4, AAP3 or AAP4 promoter.

The term hybridization, as it is used in this application, means hybridization under conventional hybridization conditions, as they are described in Sambrook et al. (Molecular Cloning. A laboratory manual, Cold Spring Harbor Laboratory Press, 2^{nd} ed., 1989), preferentially under stringent conditions. According to the present invention, hybridization also means, that, after washing for 20 minutes with 2 x SSC and 0,1 % SDS at 50 °C, preferably at 60 °C and especially preferred at 68 °C, or especially for 20 minutes in 0,2 x SSC and 0,1 % SDS at 50 °C, preferably at 60 °C and especially preferred at 68 °C, a positive hybridization signal is observed.

Hereinafter, suitable promoters for the purpose of the present invention are described.

### SUT1/SUT4 promoter

The promoter of the high affinity and low capacity sucrose transporter SUT1 is active in companion cells of the phloem. In combination with intron 2, it is also active in guard cells and in combination with intron 3 it is active only in trichomes. Generally it is more active in source tissue (e.g. adult leaves and germinating seed) than in sink tissues (e.g. meristems) or sink organs like roots, tubers, flowers, and fruits. Together with SUT4, SUT1 is responsible for phloem loading. A nucleotide sequence of the LeSUT1 promoter is given in the sequence having the SEQ ID NO: 5.

The promoter of the low affinity and high capacity sucrose transporter SUT4 is specifically active in the minor veins (phloem loading zone) of source leaves. In addition, it is very active in sink tissue. In source leaves, the SUT4 promoter can be used to drive expression of a high affinity sucrose transporter such as SUT1. This could increase sucrose loading into phloem in minor veins concomitant with an increase in sink strength in harvested organs that are sinks (e.g. fruits or seeds). A nucleotide sequence of the *Arabidopsis thaliana* SUT4 promoter is given in the sequence having the SEQ ID NO: 6.

Overexpression of the gene for the high capacity sucrose transporter SUT4 in source tissue (e.g. leaves) under control of a regulatory element according to the present invention, comprising nucleotide sequences derived from intron 2 and the SUT1 promoter, can increase sucrose loading into the phloem under conditions of high flux rates (e.g. high light intensity). This will lead to increased loading of sucrose into phloem, higher sugar content in the whole sink tissue and especially in roots, fruits, tubers, flowers and seeds, and thus higher total carbohydrate levels in harvested organs. In oil plants, this will lead to higher oil content. Number and dry weight of harvested organs such as fruits, tubers etc. will increase. Overexpression of the SUT4 gene will also be a means to manipulate flowering time and to enhance plant growth in general. What was mentioned above, will also apply to a regulatory element according to the invention with the SUT4 promoter controlling expression of the gene for the high affinity sucrose transporter SUT1.

Cosuppression or antisense suppression of SUT1 and/or SUT4 in source tissue leads to decreased sink strength and results in higher carbohydrate content in source organs. This is important to decrease competition from non-harvested sinks, and to decrease the carbohydrate investment in stems, e.g. to produce more productive dwarf varieties. In addition, this can be used to improve the sweetness of leaf crops such as lettuce and spinach. It will also lead to an accumulation of sugars in source organs and have similar effects as overexpression of SUT4 in non-vascular cells in the leaf.
Stem-specific inhibition will reduce stem length and thus increase harvest index.

Overexpression of the SUT4 gene in guard cells under the control of a regulatory element according to the present invention, comprising a nucleotide sequence from intron 2 and the SUT1 promoter, leads to changes in sugar fluxes, and thus energy and osmolyte supply can be used to increase the capacity to open or to reduce the possibility to close stomata. More open stomata will allow better influx of CO₂ for optimal photosynthesis, provided that the water supply is sufficient. It also will allow more frequent opening/closing cycles under conditions of rapidly changing light availability.

Decrease in SUT1 expression in guard cells can be achieved using the SUT1 promoter and intron 2 to express the SUT1 gene in the antisense orientation. This would block stomatal opening, e.g. during the day, and increase water use efficiency since less water is lost and thus improve plants normally not growing in dry conditions with respect to drought tolerance.

Cosuppression or antisense suppression of sucrose transporter genes will prevent efficient phloem loading and will increase sugar content in leaves. Thus the result will be bigger leaves, with more defense potential against pathogens, especially since defense genes are upregulated by increased sugar content, a thicker cuticle and more secondary metabolites, e.g. higher precursor content for production of biodegradable plastics (e.g. PHB). Both the SUT1 and SUT4 promoters alone or in combination with other cis elements, e.g. intron 2, are suitable for this purpose.

Genes under the control of a regulatory element according to the invention, comprising a nucleotide sequence derived from intron 3 and the SUT1 promoter, will be expressed exclusively in trichomes. This could be used to produce plants with modified trichomes, e.g. trichomes containing special plant products such as defense compounds (e.g. pesticides) or other compounds. This could also be used to express genes encoding transporters or other proteins in trichomes that would enhance accumulation of metals and improve the utility of transgenic plants in phytoremediation.

Overexpression of genes such as SUT1 in seeds will result in an improved germination rate. A suitable promoter is the SUT4 promoter.

Expression of both the SUT4 gene under the control of a regulatory element, comprising the SUT1 promoter and nucleotide sequences derived from intron 2, and the SUT1 gene under control of a regulatory element according to the invention with the SUT4 promoter may result in plants with very high yields.

Since higher sugar transport in the phloem is often negatively correlated with amino acid transport, overexpression of the SUT1 and/or SUT4 gene may lead to reduced amino acid content in sink tissues, e.g. in potato tubers or sugar beet tap roots. However, overexpression of amino acid transporter genes under control of a regulatory element according to the invention will result in fruits with higher amino acid and/or protein content (e.g. in soy beans and other legumes).

### SUT2 promoter

In young plants, the SUT2 promoter is active in the whole plant. In older plants, promoter activity is found in roots, in major veins of plant leaves, in sepals and anthers. A nucleotide sequence of the *Arabidopsis thaliana* SUT2 promoter is given in the sequence having the SEQ ID NO: 7.

The SUT2 promoter can be used to express plant or non-plant genes in phloem and other tissues. This could be used to increase yield or nutrient content of harvested tissues.

The SUT2 gene encodes a sucrose sensor. The SUT2 promoter can be used to decrease the expression of the SUT2 gene by expression of SUT2 in the antisense orientation or by overexpression of SUT2 leading to cosuppression. Decreased expression of SUT2 would modify sucrose sensing and sucrose transport due to SUT2 and/or other sucrose transporters (e.g. SUT1 and SUT4). Modification of sucrose transport activity could be used to increase carbon partitioning to harvested organs as suggested above.

### AAP3 promoter

The promoter of the amino acid transporter AAP3 is mainly active in the stele of roots, in flowers and in cotyledons. A nucleotide sequence of the *Arabidopsis thaliana* AAP3 promoter is given in the sequence having the SEQ ID NO: 8.

The AAP3 gene encodes an amino acid transporter. The AAP3 promoter can be used to decrease the expression of the AAP3 gene by expression of AAP3 in the antisense orientation or by over expression of AAP3 leading to cosuppression. Decreased expression of AAP3 would modify translocation of amino acids between the xylem and phloem in roots and would decrease amino acid translocation to the shoot. This can be used to decrease the concentration of toxic or undesirable nitrogenous compounds in the shoot.

### AAP4 promoter

The promoter of the amino acid transporter AAP4 is active in pollen and tapetum tissue. It is also active in major veins of the phloem of mature leaves, stem and roots. A nucleotide sequence of the *Arabidopsis thaliana* AAP4 promoter is given in the sequence having the SEQ ID NO: 9.

The AAP4 gene encodes an amino acid transporter. The AAP4 promoter can be used to express plant or non-plant genes in pollen or tapetal tissue. This could be used to generate male sterile plants or to limit cross pollination from transgenic plants (to limit transgene dissemination). Exemplary, the AAP4 promoter can be used to decrease the expression of the AAP3 gene by expression of AAP3 in the antisense orientation or by over expression of AAP3 leading to cosuppression. This could inhibit pollen function and lead to male sterile plants. This would be important for plant breeding.

A regulatory element according to the invention can be used to control the expression of a variety of plant and non-plant genes, comprising, but not restricted to, the SUT1, SUT2, SUT4, AAP3 and AAP4 gene. Thus, it can be used to influence complex transport and distribution processes, e.g. for nutritive substances like oligosaccharides and amino acids, vitamins, minerals, or growth regulatory substances such as peptide hormones or other hormones within the plant.

The invention also relates to a promoter for use in a regulatory element according to the invention, wherein the promoter is one of the SUT1, SUT2, SUT4, AAP3 and AAP4 promoters.

The invention also relates to vectors or mobile genetic elements, comprising a regulatory element according to the invention. Suitable vectors or mobile genetic elements, e.g. viruses, bacteriophages, cosmids, plasmids, yeast artificial chromosomes, T-DNA, transposable elements, insertion sequences etc., to introduce nucleotide sequences into host cells are well known to one skilled in the art of molecular cloning techniques.

Further, the invention relates to host cells like bacterial cells, yeast cells or plant cells. In a preferred embodiment of the invention, plant cells, especially of the genus *Beta vulgaris,* are transformed with a regulatory element according to the invention.

The invention also relates to plants, parts thereof, and seeds of plants, which comprise, or are derived from, cells transformed with a regulatory element according to the invention. Plants, transformed with a regulatory element according to the invention, can be elected from the group of plants comprising, but not restricted to, Pinidae, Magnoliidae, Ranunculidae, Caryophyllidae, Rosidae, Asteridae, Aridae, Liliidae, Arecidae, and Commelinidae (subclasses according to Sitte, P., Ziegler, H., Ehrendorfer, F., Bresinsky, A., eds., 1998, Strasburger - Lehrbuch der Botanik für Hochschulen; 34. ed., Gustav Fischer Verlag, Stuttgart). Thus, a transgenic plant according to the invention may be elected from the group of plants comprising, but not restricted to, sugar beet (*Beta vulgaris*), sugar cane, Jerusalem artichoke, Arabidopsis, sunflower, tomato, tobacco, corn, barley, oat, rye, rice, potato, rape, cassava, lettuce, spinach, grape, apple, coffee, tea, banana, coconut, palm, pea, bean, pine, poplar, and eucalyptus.

Further, the invention relates to a process for the production of a transgenic plant, especially of the genus *Beta vulgaris,* comprising the steps of transforming a plant cell with a regulatory element according to the invention and regenerating a plant therefrom. These steps can be performed using standard procedures.

The sequence protocol includes:
SEQ ID NO: 1: A nucleotide sequence of intron 1 of the SUT1 gene from *Lycopersicon esculentum*
SEQ ID NO: 2: A nucleotide sequence of intron 2 of the SUT1 gene from *Lycopersicon esculentum*
SEQ ID NO: 3: A nucleotide sequence of intron 3 of the SUT1 gene from *Lycopersicon esculentum*
SEQ ID NO: 4: A nucleotide sequence of a 3'untranslated region of the SUT1 gene from *Lycopersicon esculentum*
SEQ ID NO: 5: A 2,3 kb nucleotide sequence of the SUT1 promoter from *Lycopersicon esculentum*
SEQ ID NO: 6: A 3,1 kb nucleotide sequence of the SUT4 promoter from *Arabidopsis thaliana*
SEQ ID NO: 7: A 2,4 kb nucleotide sequence of the SUT2 promoter from *Arabidopsis thaliana*
SEQ ID NO: 8 A 2,5 kb nucleotide sequence of the AAP3 promoter from *Arabidopsis thaliana*
SEQ ID NO: 9 A 2,7 kb nucleotide sequence of the AAP4 promoter from *Arabidopsis thaliana*

### Brief description of drawings:

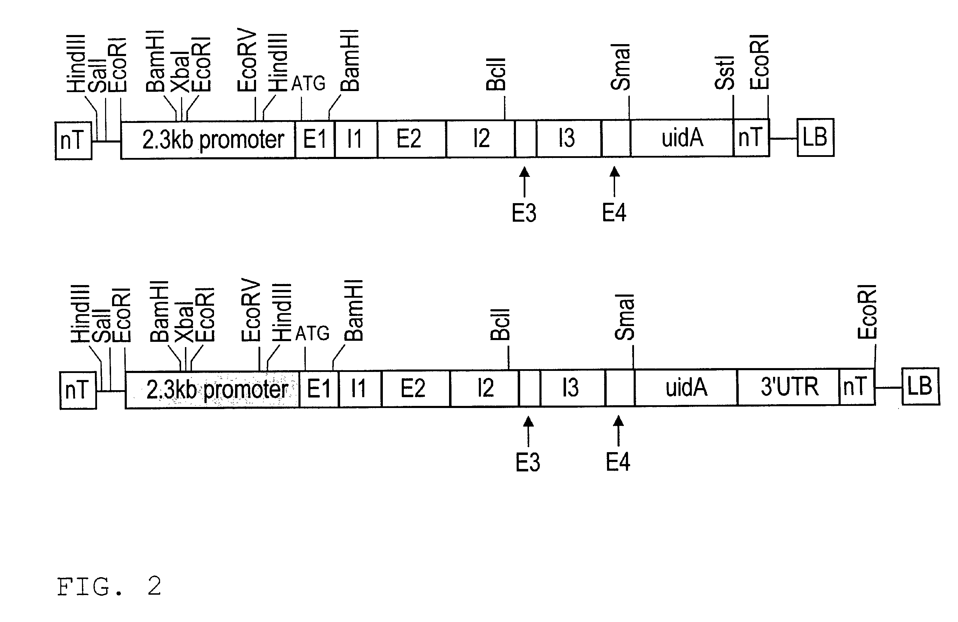

The following figures serve to elucidate the invention. It shows:

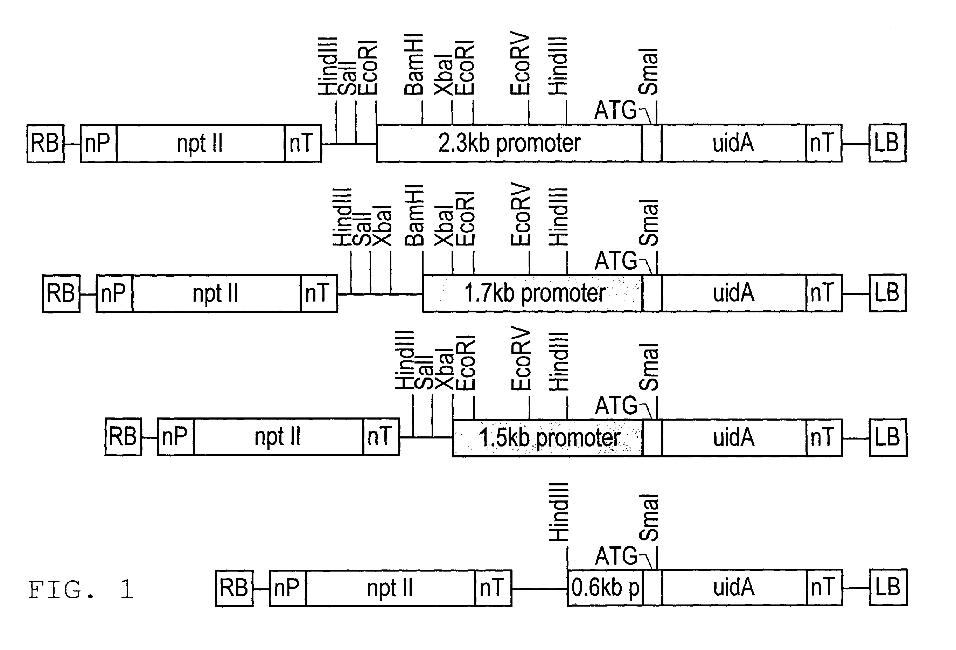

- FIG. 1: A schematic drawing of promoter deletion (promoter- GUS) constructs for LeSUT1. In this:
HindIII, SalI, EcoRI, BamHI, XbaI, EcoRV, SmaI:
Cleavage positions of restriction enzymes.
ATG: Translation start
uidA: GUS gene
npt II: Neomycin-Phosphotransferase II gene
nT: nopaline sythase terminator
nP: nopaline sythase promoter
LB: left border sequences (from T-DNA)
RB: right border sequences (from T-DNA)
kb: kilobases
p: promoter
- FIG. 2: A schematic drawing of promoter-SUT1-GUS constructs for LeSUT1. In this:
HindIII, SalI, EcoRI, BamHI, XbaI, EcoRV, SmaI,
BclI, SstI: Cleavage positions of restriction enzymes.
ATG: Translation start
uidA: GUS gene
npt II: Neomycin-Phosphotransferase II gene
nT: nopaline sythase terminator
nP: nopaline sythase promoter
LB: left border sequences (from T-DNA)
RB: right border sequences (from T-DNA)
E1, E2, E3, E4: Exons 1 to 4
I1, I2, I3: Introns 1 to 3
3'UTR: 3' untranslated region

- FIG. 3: A. Comparison of average β-glucuronidase activities of promoter-GUS, promoter-SUT1-GUS and promoter- SUT1-GUS-3'UTR constructs.
B. Percent of plants, transformed with promoter-GUS, promoter-SUT1-GUS and promoter-SUT1-GUS-3'UTR constructs, showing β-glucuronidase activity in different activity intervals.
- FIG. 4: Schematic drawing of promoter-intron-GUS-3'UTR constructs for LeSUT1
In this:
HindIII, SalI, EcoRI, BamHI, XbaI, EcoRV, XhoI SmaI:
Cleavage positions of restriction enzymes.
ATG: Translation start
uidA: GUS gene
npt II: Neomycin-Phosphotransferase II gene
nT: nopaline sythase terminator
nP: nopaline sythase promoter
LB: left border sequences (from T-DNA)
RB: right border sequences (from T-DNA)
I1, I2, I3: Introns 1 to 3
3'UTR: 3' untranslated region

The invention is further defined, by way of illustration only, by reference to the following examples.

### EXAMPLE 1

### Expression analysis of the sucrose transporter LeSUT1 by the GUS fusion system

The spatial expression within the plants was uncovered by a promoter-reporter-gene-fusion using the GUS (β-glucuronidase) gene fusion system (Jefferson et al. 1987, EMBO J. 6: 3901-3907). First, the promoter of the SUT1 gene from tomato was isolated.

For the construction of promoter-GUS constructs, the genomic clone of LeSUT1 was isolated by screening a genomic library, containing genomic DNA from *Lycopersicon esculentum* cv. VFN8 in the EMBL-3 vector (Clontech). By hybridization of a ³²P-labeled StSUT1 probe (from potato, *Solanum tuberosum*) under low stringency conditions 11 positive λ-phages could be identified. The 7 strongest hybridizing phage isolates were used to obtain λ-phage DNA by plate lysates for a restriction analysis. The 2 phages that gave the strongest signal (10/1 and 2/2) were analyzed in more detail by Southern blot analysis. A 2.1 kb BamHI fragment was isolated that, compared with the restriction pattern of the genomic clone, contains the promoter and the 5'-region of LeSUT1. This BamHI fragment was cloned into pON 184 and sequencing showed the correspondence with the 5'-end of the LeSUT1 cDNA. The translation start of LeSUT1 is at position 1693 in this fragment. The transcription start determined by comparison with the cDNA start of LeSUT1 is at position -60 of the first ATG of LeSUT1 (at position 1633 of the fragment).

For the generation of a 1.7 kb promoter-GUS construct the 2.1 kb BamHI clone was used. Because of the lack of a suitable restriction site in the N-terminal region of LeSUT1 a 840 bp fragment of the promoter was amplified by polymerase chain reaction (PCR) using the reversed primer
5'-GGGGTACCCGGGTGTACCATTCTCCATTTT-3' containing restriction sites for SmaI 15 bp downstream of the first ATG of LeSUT1 and for KpnI behind the SmaI site. The forward primer 5'-CCGATATCTCAATTGGTT-3' contained a EcoRV site which is located at position 887 in the 2.1 kb fragment. The PCR product was cloned EcoRV/KpnI into pON184 containing the 850 bp BamHI/EcoRV fragment of the 5'-end of the LeSUT1 promoter region. Afterwards the 1.7 kb promoter fragment was cloned BamHI/SmaI into the plant binary vector pBI101.3 (Jefferson et al. 1987, EMBO J. 6: 3901-3907). The reading frame of this translational fusion was checked by sequencing the uidA (GUS) gene. In this construct the first five amino acids are encoded by LeSUT1, the following seven amino acids are encoded by the polylinker of pBI101.3, followed by the uidA gene.

Expression of GUS under control of the 1.7 kb promoter fragment of LeSUT1 showed the same expression pattern, restricted to the vascular tissue in source leaves of transgenic potato and tomato plants. Unexpected in this case was the low rate of plants expressing GUS. For both species potato and tomato, the rate of transgenic plants showing GUS expression was only about 20%. This indicates a positional effect in which the promoter-GUS construct was incorporated near an enhancer. Therefore, the 1.7 kb promoter itself has low activity. In addition, the intensity of GUS derived blue staining in the veins was unexpectedly low except for one or two transgenic lines showing strong expression. Compared to Northern blot analysis showing very strong expression of SUT1 in source leaves (Riesmeier et al. 1993, Plant Cell 5: 1591-1598), the expression of GUS under the control of the SUT1 promoter was weak.

Therefore, it was tested whether an inhibiting element was present in the 1.7 kb promoter fragment or whether in the 1.7 kb fragment important upstream cis elements, responsible for the expression of SUT1, were missing. On the one hand, further upstream promoter sequences were isolated to generate a larger 2.3 kb promoter construct and on the other hand the 1.7 kb fragment was shortened to create an 1.5 kb and an 0.6 kb promoter construct (Fig. 1).

To isolate an extended promoter region of LeSUT1 a DIG-labeled probe (Boehringer) of the 5'-end of the 2.1 kb BamHI fragment was hybridized with DNA of the phage lysates 10/1 and 2/2 cut with different restriction enzymes. A positive 0.8 kb EcoRI fragment from phage 10/1 was cloned into pBlueskript SK⁺ (Stratagene). Sequencing of this fragment showed the correspondence with the 5'-end of the 2.1 kb BamHI fragment and no homology to the EMBL-3 vector.

1.5 kb promoter-GUS construct:
For the generation of the 1.5 kb promoter-GUS construct a 0.2 kb XbaI fragment from the 5'-end of the LeSUT1 promoter was deleted from the 1.7 kb promoter-GUS construct and the remaining construct was religated.

0.6 kb promoter-GUS construct:
For the generation of the 0.6 kb promoter-GUS construct a 1.1 kb HindIII fragment from the 5'-end of the LeSUT1 promoter was deleted from the 1.7 kb promoter-GUS construct and the remaining construct was religated.

2.3 kb promoter-GUS construct:
For the generation of the 2.3 kb promoter-GUS construct the 0.8 kb EcoRI fragment of the extended 5'-region of the LeSUT1 promoter was cloned into the EcoRI site of pBlueskript SK⁺. This fragment was then cut with SalI/XbaI and cloned into the SalI/XbaI digested 1.7 kb promoter-GUS construct.

The expression of these promoter-GUS constructs was investigated in *Nicotiana tabacum*. The specificity of promoters in different plant species are maintained.

The translational promoter-GUS fusions were introduced into the genome of *Nicotiana tabacum* via *Agrobacterium* mediated gene transfer. After selective regeneration of plants on kanamycin containing media leaf discs of plants from sterile culture were tested for β-glucuronidase (GUS) activity.

In Table 1, the rates of plants showing β-glucuronidase activity is presented. For the 2.3 kb, 1,7 kb and 0.6 kb promoter constructs the rates are similar between 40-50 %. Expression was lowest in plants containing the 1.5 kb promoter construct. The intensity of average staining was very low for all constructs after 2 days of incubation in the X-Gluc solution. The pattern of the weak blue staining was in most cases restricted to the major veins for all four constructs.

### Influence of intragenic sequences (introns) and the 3'UTR of LeSUT1 on the expression of GUS

None of the deletion constructs resulted in expected high expression rates and levels of the β-glucuronidase. The absence of required further upstream promoter sequences is unlikely. Most plant genes require shorter promoters for their expression. More likely is that in the case of LeSUT1 other sequences downstream of the translation start are required for sufficient gene expression.

To figure out if intragenic sequences play a role in SUT1 expression two further constructs were generated (Fig. 2). In

**Table 1**

| β-glucuronidase (GUS) activity in transgenic LeSUT1 promoter-GUS tobacco lines (after 2 days incubation in X-Gluc solution). | | | |
|---|---|---|---|
| Construct | No. of transgenic plants | No. of plants showing GUS-staining | % of plants showing GUS-staining |
| 2.3P-GUS | 41 | 16 | 39 |
| 1.7P-GUS | 36 | 14 | 39 |
| 1.5P-GUS | 31 | 5 | 16 |
| 0.6P-GUS | 36 | 19 | 50 |

the one construct the longest promoter fragment (2.3 kb) together with the entire coding region (including introns) of LeSUT1 ending 6 amino acids before the stop codon was fused to the GUS gene. LeSUT1 has four exons and three introns. The second construct contains in addition an approx. 1.2 kb fragment containing the 3'UTR of LeSUT1 placed between the GUS gene and the nopaline synthase terminator (Fig. 2).

2.3 kb promoter-SUT1-GUS construct:
For the generation of a construct that, in additionally to the promoter region, contains the whole genomic sequence of LeSUT1, a genomic 7.1 kb EcoRI fragment containing 1.5 kb of the promoter region, the whole genomic sequence (4 exons and 3 introns) of the SUT1 gene and further upstream sequence was cloned into pBlueskript SK⁺ with the promoter sequence orientated to the SalI site of the polylinker of the vector. Because of a lacking suitable restriction site around the stop codon of SUT1 a 1.4 kb fragment was amplified by PCR. The reversed primer LeSUT1-1394rev (5'-GAAACCGCCCATCCCGGGTGGTGGTTTAG-3') contained a SmaI site 18 bp in front of the SUT1 stop codon, the forward primer LeSUT1-1394for (5'-GTGGGCTTGTAAACGGTTGTAAGTCAC-3') was designed in the middle of the second intron 357 bp in front of a BclI site at position 2764 behind the first ATG of SUT1. This PCR product was then cut with BclI and SmaI and cloned into the 7.1 kb EcoRI fragment in pBlueskript SK⁺ that had been digested with BclI and SmaI. Afterwards, to this clone, the 0.8 kb EcoRI fragment containing the 5'-end of the LeSUT1 promoter region was cloned into the EcoRI site. The final 6.0 kb fragment containing 2.3 kb of the promoter region and the genomic sequence ending 6 amino acids before the stop codon of LeSUT1 was then cloned into the SalI and SmaI digested plant binary vector pBI101.3 (Jefferson et al. 1987, EMBO J. 6: 3901-3907). The maintenance of the reading frame was checked by sequencing into the SmaI fusion site by using the primer 5'uidA.

2.3 kb promoter-SUT1-GUS-3'UTR construct:
The 3'UTR of LeSUT1 was isolated by PCR using the forward primer 5'-TTCCGGCCGAAAAAATTACAAAAGACGAGGAAG-3' containing a EagI site and the reversed primer
5'-TACCGAGCTCCTAGGCGAGGTCGACGGTAT-3' containing a SalI site on the 7.1 kb genomic EcoRI clone of LeSUT1. The 1.2 kb product was cut with EagI/SalI and cloned into pBlueskript SK⁺. To minimize possible PCR errors approximately 1 kb of the C-terminal sequence of the PCR-product in pBlueskript SK⁺ was replaced by genomic sequence from the 7.1 kb EcoRI clone by using BstI that cuts at position 212 of the 3'UTR and SalI that cuts upstream of the 7.1 kb EcoRI fragment in the pBlueskript SK⁺ vector. The fragment was then cut out with EagI and EcoRV. The 5'overhang of the EagI site was filled in with the Klenow enzyme. This fragment with blunt ends was then cloned into the SstI site between the uidA gene and the nos terminator of pBI101.3. The 6.0 kb fragment, containing 2.3 kb of the promoter region and the genomic sequence ending 6 amino acids before the stop codon of LeSUT1 was then cut with SalI and SmaI and cloned into pBI101.3 containing the 3'UTR.

### Quantitative effects of intragenic sequence and the 3'UTR of LeSUT1 on β-glucuronidase activity in Nicotiana tabacum

The GUS fusions were introduced into the genome of *Nicotiana tabacum* and β-glucuronidase activity was first analyzed in transgenic tobacco plants from sterile culture. For the 2.3P-SUT1-GUS construct 20 transgenic plants were obtained of which 11 plants showed GUS expression. These 55% of transgenic plants showed a clearly stronger and more specific blue staining on average than the plants transformed with any of the promoter deletion constructs. For the 2.3P-SUT1-GUS-3'UTR construct 46 transgenic plants were obtained of which 36 (78%) plants showed GUS expression. The expression level determined as intensity of blue staining in these plants appeared to be the highest on average.

To quantify the visual impression of higher GUS expression for these constructs the activity of the β-glucuronidase enzyme in the transgenic plants was determined (Jefferson et al. 1987, EMBO J. 6: 3901-3907). For this, all transgenic tobacco lines of the 2.3P-GUS, the 2.3P-SUT1-GUS and the 2.3P-SUT1-GUS-3'UTR constructs were transferred into the greenhouse to grow them under more physiological conditions compared to those in sterile culture. After 10-12 weeks after transfer into the greenhouse fully expanded big source leaves were cut of the plant approx. 1 cm below the leaf base in the petiole and frozen in liquid nitrogen. To achieve an equal distribution of vascular tissue and leaf mesophyll tissue the leaves were first ground in liquid nitrogen to a fine powder. Aliquots of this powder were then used to determine the β-glucuronidase activity in the leaves using the standard fluorimetric GUS assay (Gallagher, S.R., 1992, in GUS protocols: Using the GUS gene as a reporter of Gene expression, Gallagher, S.R., (ed.), Academic Press, Inc., pp 47-59; Jefferson et al. 1987, EMBO J. 6: 3901-3907).

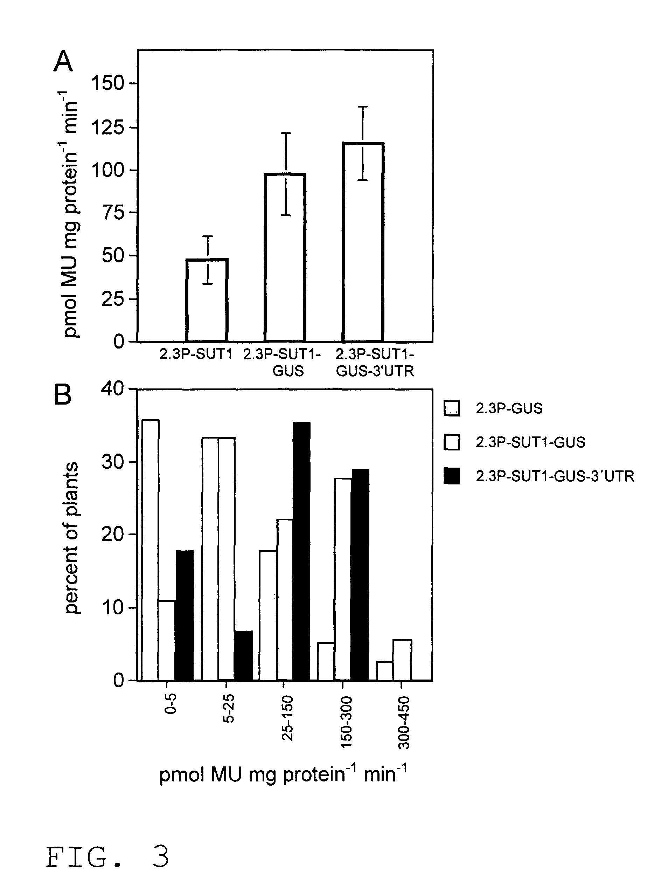

The activity was determined as the average activity of the β-glucuronidase (Fig. 3A) for all plants and the values were separated in percent of plants showing enzyme activity in different activity intervals (Fig. 3B).

The activity of the β-glucuronidase was nearly 50 pmol MU mg protein⁻¹ min⁻¹ for the 2.3P-GUS construct (Fig. 3A). The activity of GUS in plants containing the 2.3P-SUT1-GUS construct was about two-fold higher in average and the highest activity in average could be observed for the 2.3P-SUT1-GUS-3'UTR construct with an activity of about 120 pmol MU mg protein⁻¹ min⁻¹. The higher activity of this construct compared with the 2.3P-SUT1-GUS construct is not significant (t-test, α = 0.05). Nevertheless, visually the GUS expression appeared to be stronger on average.

The distribution of β-glucuronidase activities into different intervals shows that the vast majority (70% of all plants) of the 2.3P-GUS plants have enzyme activities in a range of 0-25 pmol MU mg protein⁻¹ min⁻¹. The distribution of the 2.3P-SUT1-GUS plants is clearly shifted to a majority of plants showing activity between 5 and 150 pmol MU mg protein⁻¹ min⁻¹ (56% of all plants). The clear majority of the 2.3P-SUT1-GUS-3'UTR plants show an activity of 25-300 pmol MU mg protein⁻¹ min⁻¹ (65% of all plants).

This distribution of the different enzyme activities for the different constructs indicate both an enhancing effect of the exons and/or introns and the 3'UTR on the activity of the LeSUT1 promoter.

### Histochemical analysis of heterologous expression of the 2.3P-SUT1-GUS-3'UTR constructs in Nicotiana tabacum

The expression pattern in the 2.3P-SUT1-GUS and the 2.3P-SUT1-GUS-3'UTR tobacco lines were similar. Of 36 2.3P-SUT1-GUS-3'UTR lines 34 showed the same expression pattern with varying intensities. 2 plants showed a different staining pattern. The 11 2.3P-SUT1-GUS lines showed the same average expression pattern as the 3'UTR construct with lower intensity in general.

The expression pattern in leaves of tobacco plants grown in sterile culture was clearly different compared with plants expressing GUS under the control of the 2.3 kb promoter fragment only. GUS expression was observed in all leaf veins, in trichomes and in guard cells. The staining in the minor veins was not equally distributed within the vein but appeared to be concentrated in spots. The expression in leaves of plants grown in the greenhouse was the same.

The GUS-expression in the vascular tissue was further investigated in thin sections to determine in which cell type the GUS expression is localized. GUS staining was detectable in the internal phloem and stronger in the external phloem of mid veins. In longitudinal midvein sections the staining appeared to be concentrated in spots along the phloem. In cross sections of minor veins GUS expression was clearly detectable in companion cells. In sieve elements of minor veins no GUS expression was observed. In longitudinal sections of midveins GUS expression was strongest in companion cells, concentrated around the nuclei. In some cases little staining was also detectable in sieve elements.

### Histochemical analysis of heterologous expression of LeSUT1 2.3P-intron-GUS-3'UTR constructs in Nicotiana tabacum

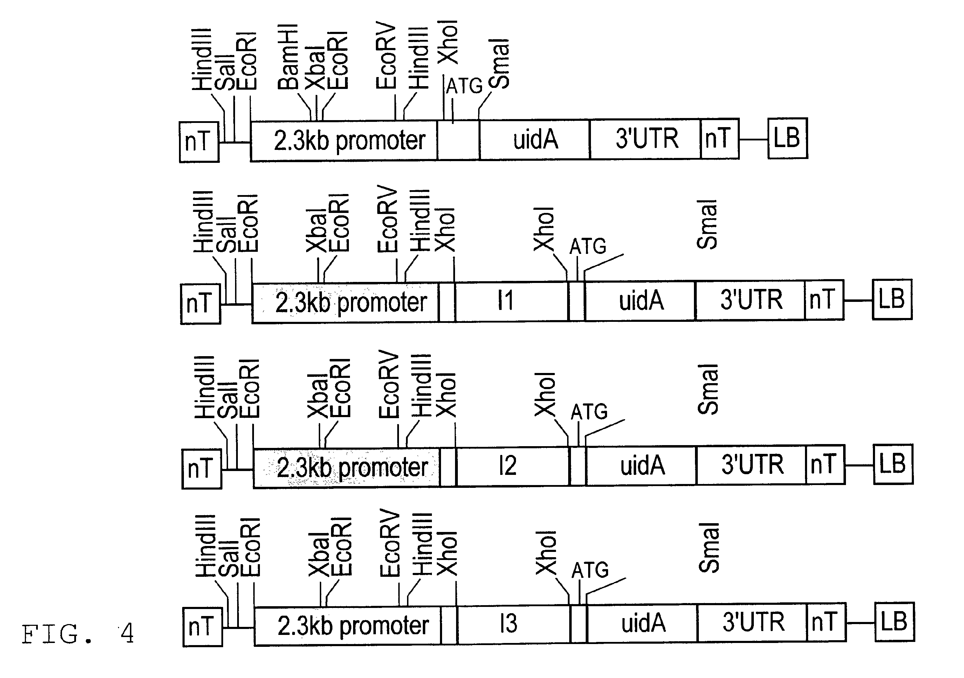

To reveal the function of the introns on LeSUT1 expression, further constructs were made (Fig. 4). To allow a high efficiency of intron splicing the 5'UTR was chosen for the intron insertion. To maintain the enhancing effect of the 3'UTR, the 3'UTR was kept in the intron constructs between the GUS gene and the nopaline synthase terminator. The introduction of the necessary XhoI restriction site and the maintenance of the intron border sequences resulted in little sequence modifications within the 5'UTR of LeSUT1.

To insert introns into the 5'UTR of LeSUT1 a restriction site was introduced for XhoI which does not cut in any of the introns, the plant binary vector, the 2.3 kb promoter fragment, or the 3'UTR of LeSUT1. Using PCR, a 650 bp fragment of the 3'end of the 2.3 kb promoter was amplified using the reversed primer LeSUT1PXhoIrev
(5'-TTTCCCGGGTGTACCATTCTCCATTTTTTTTTCTTCTAAGAAACTAAAATTGCTCGA GTTTAATTTTGGG-3') containing the previously introduced SmaI site and, further upstream, a XhoI site leading to an exchange of three basepairs and the forward primer LeSUT1PXhoIfor (5'-GATAAATCAAGGTGATATATGTACATAC-3') containing an endogenous Bsp4107I site which cuts twice in the uidA gene. Therefore, the PCR product was then used in a triple ligation step together with the excised 5'SalI/Bsp1407I fragment of the 2.3 kb promoter fragment and the SalI/SmaI cut pBI101.3 vector containing the 3'UTR of LeSUT1.

The three introns were amplified using PCR. The following primers, containing XhoI sites at both ends, were used:

For Intron 1:
LeSUT1intron1for    5'-GCTAATAACATGCTCGAGGTAATTTTCAAATCG-3'
LeSUT1intron1rev    5'-CCAGTAGTGCTCTGCTCGAGCCCTG-3'

For Intron 2:
LeSUT1intron2for    5'-CCCTTGGTATTCCTCGAGCGGTGAGTTTC-3'
LeSUT1intron2rev    5'-CCAAAGCAAATGGAATACCTCGAGTTACCTG-3'

For Intron 3:
LeSUT1intron3for    5'-CTTGCAATTGTTGTACTCGAGGTAC-3'
LeSUT1intron3rev    5'-CTTACTAGTGACCTCGAGATCTATATC-3'

The introns were then cloned into the XhoI digested 5'UTR of the 2.3PXhoI-GUS-3'UTR construct. The correct orientation of the introns was proven by sequencing.

In addition to the intron constructs the construct with the XhoI modified 5'UTR was also introduced into the genome of tobacco plants as a control. For the intron 1 construct 30 kanamycin resistant plants were obtained, whereas 8 were obtained for the intron 2 construct, 20 were obtained for the intron 3 construct, and 24 were obtained for the control construct. Leaves from sterile culture grown plants were incubated over night in X-Gluc solution at 37 °C.

17 plants containing the intron 3 construct showed GUS activity (= 85%) of which in 16 plants blue staining could be exclusively detected in the trichomes. One plant showed an overall GUS expression in the leaf. Six plants containing the intron 2 construct showed GUS expression (= 75%). The expression in 6 of these plants was restricted to the guard cells and veins. Two plants showed no staining.

In seven tobacco lines (= 30%) transformed with the control construct weak, non-specific GUS staining was observable. In general, the staining pattern was similar to those plants that had been transformed with the 2.3 P-GUS construct.

The results indicate a clear enhancing and/or spatial regulation function for intron 3 and intron 2. The presence of intron 3 is necessary to drive high level gene expression in trichomes whereas intron 2 represents the element required for gene expression in veins and guard cells.

Intron 1 caused a negative effect on gene expression. Of 24 plants transformed with the control construct, only 7 (30%) showed GUS staining. However, none of the plants (of 30) containing the Intron 1 construct showed visible GUS staining. This indicates, that intron 1 has a negative regulatory effect on gene expression. Therefore, the introduction of intron 1 into other genes, particularly into existing introns may down-regulate expression. This represents a novel strategy to regulate expression.

The comparable expression of the control construct to the 2.3P-GUS constructs indicates that at least the modification of the 5'UTR by introducing the XhoI site did not affect the expression level. Furthermore, this result indicates that the 3'UTR is not sufficient, in combination with the promoter, to provide strong expression observed for the 2.3P-SUT1-GUS-3'UTR construct.

### EXAMPLE 2

### Expression analysis of the sucrose transporter gene SUT2 by promoter-GUS fusion in Arabidopsis

The promoter of AtSUT2 was isolated by polymerase chain reaction (PCR) using Pfu-polymerase and the primers AtSUT2Pfor (5'-ACGCTTGTCGACCCGGCTCTATCACGTTAACAC-3' and 5'-ACGCTTGTCGACCGTTTGAGAAATGACGAAGGAG-3' for the 2.2 kb and 1.2 kb promoter fragments, respectively) and AtSUT2Prev (5'-GTCCCCCGGGCAACACACAGATCCCTAATTCG-3') on genomic DNA of *Arabidopsis thaliana* Col-O ecotype. Transcriptional fusions were generated by cloning 2.2 kb and 1.2 kb promoter fragments into the SalI/SmaI site of pGPTV-HPT (Becker et al. 1992, Plant Mol. Biol. 20: 1195-1197). *Arabidopsis* was transformed by vacuum infiltration using transformed *Agrobacterium* GV2260 (Clough and Bent 1998, Plant J. 16: 735-743). Among 10 hygromycin resistant transformant lines for the 1.2 kb promoter construct 9 showed the same expression pattern. For the 2.2 kb promoter construct 2 plants were regenerated both showing the same expression as the 1.2 kb promoter constructs.

In young seedlings and plants GUS expression was found in all tissues of the shoot and roots. In older greenhouse grown plants GUS expression in source leaves was restricted to the major veins and hydathodes. In stem, no GUS expression was detectable. In flowers, GUS expression was detected in sepals, anthers, the stigma of the pistil and at the peduncel.

### EXAMPLE 3

### Expression analysis of AtSUT4 in Arabidopsis by promoter-GUS fusions

To analyze the expression of AtSUT4 in plants, the promoter was isolated and fused to the GUS reporter gene (Jefferson et al. 1987, EMBO J. 6: 3901-3907). A 3.1 kb, a 2.2 kb and a 1.1 kb fragment was isolated by polymerase chain reaction on genomic DNA of *Arabidopsis thaliana* Col-O using Pfu polymerase and transcriptionally fused to the GUS gene in the plant binary vector pGPTV-HPT (Becker et al. 1992, Plant Mol. Biol. 20: 1195-1197). For the isolation of the promoter fragments the following oligonucleotides were used as primers:

AtSUT4Prev 5'-TCCCCCGGGCTCGCTTCACAGTCGTCGTGGCGTAG-3'
AtSUT4P3.1kbfor 5'-GTTTGTTGTCGACGGGCGAAATCTCGCATAACTTC-3'
AtSUT4P2.2kbfor 5'-ACGGTCGACAGGGTCGCATCTCGATATTATGG-3'
AtSUT4P1.1kbfor 5'-ACGCTTGTCGACGACCCGGTGAGTAATTGAACGC-3'

For the 3.1P-GUS construct, 17 plants could be regenerated on hygromycin containing media of which 14 showed an identical GUS expression, for the 2.2P-GUS construct 12 plants were obtained of which 8 showed an identical expression of GUS.
The expression pattern for the 3.1P-GUS and the 2.2P-GUS constructs were the same. No transformants were obtained for the 1.1P-GUS construct.

In germinating seed and in very young seedlings an overall expression of GUS was found. In the developing young plant the staining was more specific, with highest activity in the center of the rosette and sink leaves, and after transition to source leaves expression was restricted to the minor veins. This expression pattern is in contrast to LeSUT1 from tomato, which drives expression in all veins of source leaves.

In the inflorescence, GUS activity was found within developing flowers in the anther and pistil. At anthesis, staining was restricted to the anthers. In mature flowers GUS activity was hardly detectable. After pollination GUS activity was found throughout the developing silique and was relatively strong in the funiculi and the ingrown pollen tubes.

In general, GUS activity in flowers and fruits was restricted to the growing organs still in development without high specificity, indicating a role for AtSUT4 in growing sink organs for direct sucrose uptake into sink cells.

GUS expression was never found in stems except in the axils of branches. GUS activity remained strongest throughout development in the center of the plant rosette.

GUS expression in roots was strongest at the point of lateral root initiation. GUS activity was also detectable in the vascular system with stronger activity at the branches of lateral roots.

### EXAMPLE 4

### Expression analysis of AtAAP3 in Arabidopsis by promoter-GUS fusions

To investigate the expression of AAP3 pattern, a 7 kb AAP3 promoter was fused to the GUS gene and introduced into tobacco (Fischer et al. 1995, J. Biol. Chem. 270: 16315-16320, Fischer 1997, Dissertation, Eberhard-Karls University, Tuebingen). However, the expression of GUS activity was not stable, most of the transformed lines showed no staining at all. This might be due to the length of the promoter used. Therefore, two promoter-GUS fusion constructs which carry 1.5 kb and 3.5 kb of the AAP3 promoter were constructed. *Arabidopsis* and *Nicotiana* plants were transformed with these constructs. Transgenic lines were obtained and analyzed from 1.5 kb (1.5P-GUS) and 3.5kb (3.5P-GUS) promoter-GUS fusion constructs, respectively. In addition, 2.5 kb of the nucleotide sequence upstream from the transcriptional initiation was sequenced.

GUS activity in seedlings from the transformed lines of *Arabidopsis thaliana* was mainly observed in the root vascular tissue, cotyledons and the tip of the stamen. Both the 1.5P-GUS plants and 3.5P-GUS plants showed GUS activity in root vascular tissue. However, compared to the lines of 3.5P-GUS plants the lines of 1.5P-GUS plants showed stronger activity in root vascular tissue. To investigate the tissue specificity in detail, the roots from 3.5P-GUS plants were embedded in resin. Cross section of the roots showed GUS staining in the cells of central cylinder. No staining was observed in the xylem. The GUS activity was also observed in flowers: at the tip of the filament. For this pattern of expression, no significant difference between the 1.5P-GUS and the 3.5P-GUS plants was detected. Interestingly, GUS activity in stamen was not found in earlier developmental stages. It was visible only after the stage 13 according to Smyth's classification.

In *Arabidopsis*, dehiscence usually takes place at stage 13, just before anthesis. Cross section of a flower at stage 12 embedded in a resin showed GUS activity in the filament of the stamen which is not dehisced. This result indicated that the expression is induced slightly before the dehiscence.

Tobacco *(Nicotiana tabacum)* plants were transformed by the leaf disc method with *Agrobacterium.* 110 lines for the 1.5P-GUS construct and 76 lines for the 3.5P-GUS were obtained. Roots from both lines (13 of 1.5P-GUS line, 5 of 3.5P-GUS line) were stained. As seen in *Arabidopsis* plants, different GUS activities between the two constructs were also observed in roots of tobacco lines. 1.5P-GUS plants did not show any staining in roots even when they were incubated in ferro/ferri cyanide 3 mM/0.5 mM condition overnight. On the other hand, all the lines of 3.5P-GUS plants showed staining in the vascular strand. For detail analysis, the stained roots were embedded in resin. GUS staining was localized in the phloem of the roots.

Transformed *Arabidopsis* and tobacco plants expressing 3.5P-GUS showed stronger GUS activity in vascular tissue of roots than the 1.5P-GUS plants. Therefore, it might be concluded that the 3.5 kb promoter has enhancer motifs that are not present in the 1.5 kb promoter-GUS region.

AAP3 promoter-GUS studies revealed that AAP3 is expressed in the vascular tissues of *Arabidopsis* roots. In addition, in 3.5P-GUS tobacco plants, GUS activity was found in the phloem. It indicates its role in loading of amino acids into the phloem.

The promoter-GUS assay of AAP3 also demonstrated that AAP3 is specifically expressed in the connective tissues of stamen. Interestingly, the expression of the reporter gene was induced just slightly before dehiscence. Dehiscence is generally regarded as a desiccation process. More likely, dehiscence is a more finely regulated process than being just a consequence of desiccation. The regulation may include transcriptional control of some genes. It might be that accumulation of osmotically active compounds, such as sugars, proline and betaine is accelerated and cause water efflux from the anther wall and then triggers the dehiscence. Considering that AAP3 recognizes proline and other compatible solutes, which are known as major osmolytes in plants, it may also play a role in accumulating osmotically active components in connective tissue and triggering the dehiscence. AAP3 may also recover amino acids from dehisced anthers which might not require amino acids any more.

### EXAMPLE 5

### Expression analysis of AtAAP4 in Arabidopsis by promoter-GUS fusions

To study the expression pattern of AAP4, a 2.5 kb promoter region was isolated by polymerase chain reaction on *Arabidopsis* genomic DNA using Pfu-polymerase (Stratagene, USA). *Arabidopsis* plants were transformed and analyzed for GUS expression.

68 transformants (2 lines from pGPTV-HPT vector, 66 lines from pGPTV-BAR vector) were obtained. Organs from those transformants were harvested and stained in the ferro/ferri cyanide concentration of 3mM/0.5mM overnight. The GUS staining was mainly observed in vascular tissues of the leaves, anthers, roots and sepals of mature flowers.

The GUS-staining was observed in the vascular tissues of leaves. No GUS activity was detected in vascular tissues of young leaves. In middle-sized leaf, the GUS activity was found in the major veins on the tip of the leaf. Therefore, the expression of the fusion gene seemed to be developmentally upregulated and follows sink-source transition. Sections of the mature leaves revealed the GUS-staining in phloem. Vascular tissues of sepals and petals showed GUS activity. Anther tissues showed strong GUS activity. The GUS activity was already observed in earlier stages of development. In the later stages of flower development, anther tissues did not show GUS staining any more. However, pollen grains released from mature flowers showed weak staining.

To investigate the tissue expressing GUS gene, young flowers with strong staining in anther tissue were embedded in resin and sectioned. The GUS staining was found both in pollen grains and the tapetum tissue. Tapetum tissues seemed to be GUS-active throughout their existence. The GUS-expression in pollen grains seemed to be more complicated. Tetrad microspores showed staining. GUS activity was not observed at the later stage of development. However, pollen grains released from mature anthers showed GUS activity. Root vascular tissue was GUS-active in all three lines tested, the GUS activity was restricted to the vascular tissue.

*Arabidopsis* plants which express the AAP4-GUS fusion gene showed GUS activity in pollen and tapetum tissue. Promoter analysis of the AAP4 gene revealed that it has a high homology to the minimal pollen-specific regulatory element found in tomato lat52 promoter. As was the case in the lat52 promoter, it may be that some unknown domains of the AAP4 promoter are cooperating with the pollen-specific-element-like sequence to regulate AAP4 expression in pollen. In addition, lat52 promoter-GUS studies showed only a weak expression in the tapetum tissue. Therefore a novel cis-element might exist in the AAP4 promoter which confers the expression in the tapetum tissue.

*Arabidopsis* plants which express the AAP4 promoter-GUS fusion gene showed GUS activity in the major veins of mature leaves. Microscopic analysis revealed that the GUS staining is restricted to the phloem, indicating AAP4 expression in the phloem. AAP4 might also be functional at the phloem/xylem border, transporting amino acids from the xylem into the phloem.

AAP4 expression was detected in pollen grains and tapetum tissues of the AAP4 promoter-GUS plants. Because of its rapid maturation, pollen might require a large amount of nutritional amino acids. Furthermore, certain amino acids like proline are known to be accumulated in the pollen, probably as osmolytes. Therefore, the developing pollen grains can be the major amino acid sink in the whole plant. Therefore, AAP4 can is an excellent candidate responsible for the amino acid loading into pollen grains.

The tapetum tissues also showed strong GUS activity in AAP4 2.5kb promoter-GUS plants. The tapetum tissue consists of cytosol-rich cells whose primary function is nutritive and it is formed from primary walls by repeated vertical cell division. Therefore, the tapetum tissue may require nutritive amino acids to sustain the development, and AAP4 may be importing amino acids in the tapetum tissues.

## Claims

1. Regulatory element for the modification of gene expression in transgenic plants, comprising a promoter and a nucleotide sequence, wherein said nucleotide sequence is a nucleotide enhancer and/or repressor sequence comprising a non-coding region of a sucrose transporter gene.

2. Regulatory element according to claim 1, wherein said nucleotide enhancer and/or repressor sequence is derived from *Lycopersicon esculentum*.

3. Regulatory element according to claim 1 or 2, wherein said nucleotide repressor sequence is derived from intron 1 of the SUT1 gene.

4. Regulatory element according to one of claims 1 to 3, wherein said nucleotide enhancer sequence is derived from intron 2 and/or intron 3 of the SUT1 gene.

5. Regulatory element according to claim 3, wherein said nucleotide repressor sequence comprises the sequence of SEQ ID NO: 1, or the complementary sequence of SEQ ID NO: 1, or hybridizes with the nucleotide sequence of SEQ ID NO: 1.

6. Regulatory element according to claim 4, wherein said nucleotide enhancer sequence comprises the sequence of SEQ ID NO: 2, or the complementary sequence of SEQ ID NO: 2, or hybridizes with the nucleotide sequence of SEQ ID NO: 2.

7. Regulatory element according to claim 4, wherein said nucleotide enhancer sequence comprises the sequence of SEQ ID NO: 3, or the complementary sequence of SEQ ID NO: 3, or hybridizes with the nucleotide sequence of SEQ ID NO: 3.

8. Regulatory element according to one of claims 1 to 7, further comprising a nucleotide sequence of the 3' untranslated region of the SUT1 gene.

9. Regulatory element according to claim 8, wherein the nucleotide sequence of the 3' untranslated region comprises the nucleotide sequence of SEQ ID NO: 4, or the complementary nucleotide sequence of SEQ ID NO: 4, or hybridizes with the nucleotide sequence of SEQ ID NO: 4.

10. Regulatory element according to one of claims 1 to 9, wherein said promoter is one of the promoters of the SUT1, SUT2, SUT4, AAP3 or AAP4 genes.

11. Regulatory element according to claim 10, wherein said promoter comprises,
a) the nucleotide sequence of SEQ ID NO: 5, SEQ ID NO: 6, SEQ ID NO: 7, SEQ ID NO: 8, or SEQ ID NO: 9, or
b) a nucleotide sequence complementary to the nucleotide sequence of SEQ ID NO: 5, SEQ ID NO: 6, SEQ ID NO: 7, SEQ ID NO: 8, or SEQ ID NO: 9, or
c) a nucleotide sequence hybridizing with a nucleotide sequence of SEQ ID NO: 5, SEQ ID NO: 6, SEQ ID NO: 7, SEQ ID NO: 8, or SEQ ID NO: 9.

12. Promoter for use in a regulatory element according to one of claims 1 to 11, wherein said promoter is one of the promoters elected from the group of promoters comprising the SUT1, SUT2, SUT4, AAP3 and AAP4 promoter.

13. Promoter according to claim 12 for use in a regulatory element according to one of claims 1 to 11, wherein said promoter comprises
a) the nucleotide sequence of SEQ ID NO: 5, SEQ ID NO: 6, SEQ ID NO: 7, SEQ ID NO: 8, or SEQ ID NO: 9, or
b) a nucleotide sequence complementary to the nucleotide sequence of SEQ ID NO: 5, SEQ ID NO: 6, SEQ ID NO: 7, SEQ ID NO: 8, or SEQ ID NO: 9, or
c) a nucleotide sequence hybridizing with a nucleotide sequence of SEQ ID NO: 5, SEQ ID NO: 6, SEQ ID NO: 7, SEQ ID NO: 8, or SEQ ID NO: 9.

14. Vector or mobile genetic element, comprising a regulatory element according to one of claims 1 to 11.

15. Host cell, comprising a vector according to claim 14.

16. Host cell, wherein the host cell is a bacterial cell, a yeast cell or a plant cell.

17. Plants and parts thereof, transformed with a regulatory element according to one of claims 1 to 11.

18. Transgenic plant according to claim 17, wherein said plant is elected from the group of plants comprising Pinidae, Magnoliidae, Ranunculidae, Caryophyllidae, Rosidae, Asteridae, Aridae, Liliidae, Arecidae, and Commelinidae.

19. Transgenic plant according to claim 17 or 18, wherein said plant is elected from the group of plants comprising sugar beet (Beta vulgaris), sugar cane, Jerusalem artichoke, Arabidopsis, sunflower, tomato, tobacco, corn, barley, oat, rye, rice, potato, rape, cassava, lettuce, spinach, grape, apple, coffee, tea, banana, coconut, palm, pea, bean, pine, poplar, and eucalyptus.

20. Seeds of plants according to one of claims 17 to 19.

21. Use of a nucleotide enhancer sequence as disclosed in claims 1 to 11 to enhance a promoter.

22. Use of a nucleotide repressor sequence as disclosed in claims 1 to 11 to inhibit a promoter.

23. Use of a regulatory element according to one of claims 1 to 11 to enhance or inhibit expression of a plant gene.

24. Use according to claim 23 to enhance or inhibit expression of a SUT1, SUT2, SUT4, AAP3 or AAP4 gene.

25. Use according to one of claims 23 or 24 to enhance carbon dioxide uptake in plants.

26. Use according to one of claims 23 or 24 to enhance plant growth.

27. Use according to one of claims 23 or 24 to enhance sucrose phloem loading capacity in plants.

28. Use according to one of claims 23 or 24 to increase sugar content in plant tissues or organs.

29. Use according to one of claims 23 or 24 to increase amino acid and/or protein content in plant tissues or organs.

30. Use according to one of claims 23 or 24 to increase oil content in plant tissues or organs.

31. Process for the production of a transgenic plant, especially of the genus *Beta vulgaris,* comprising the steps of transforming a plant cell and regenerating a plant therefrom, wherein said plant cell is transformed with a regulatory element according to one of claims 1 to 11.

32. Process according to claim 31 for the production of a transgenic plant, wherein at least one of the SUT1, SUT2, SUT4, AAP3 or AAP4 genes is expressed under the control of a regulatory element according to one of claims 1 to 11.
